# EUROPEAN PATENT SPECIFICATION

(11) **EP 1 736 183 B1**
(45) Date of publication and mention of the grant of the patent: **21.01.2009**
(21) Application number: 06253300.5
(22) Date of filing: 26.06.2006
(51) Int. Cl.: A61M 1/00

(54) **A medical suction disposal system liner**
Einlage für ein medizinisches Absaug- und Entsorgungssystem
Sachet jetable pour un système d'aspiration médicale

(30) Priority: 25.06.2005 GB 0512975
(43) Date of publication of application: 27.12.2006
(73) Proprietor: VACSAX Limited, Plymouth, PL7 5BG (GB)
(72) Inventor: Hodgkins, Stephen William, Ivybridge Devon, PL21 0XN (GB); Saunders, Robert Alan, Plymouth Devon PL2 1QG (GB)
(74) Representative: Greenwood, Matthew David

(56) References cited:
- DE-U1- 9 210 829
- GB-A- 2 333 459
- GB-A- 2 401 775
- US-A- 5 533 638

## Description

The present invention relates generally to a medical suction disposal system and particularly to a liner for use in such a system. Medical suction disposal systems are used, for example, in hospital operating theatres and accident and emergency departments to remove and contain medical waste so that it can be disposed of safely.

It is known to use re-useable rigid jars, formed from glass or moulded plastics material, as the receptacle for the medical waste. However, disposable systems are preferred and more recently vacuum jars have been used in conjunction with disposable liners. The liner (also known as a liner bag) is fitted with a cap having suction line and vacuum connections and itself forms the receptacle for medical waste; the contents of the liner are subsequently disposed of, usually by incineration.

It is known to provide a liner in the form of a flexible bag which fits into a solid vacuum jar. However, if the bag is too flexible, although it can be easily packaged for transport, the bag will not be self-supporting and will not stand up by itself. This is a particular problem when the bag is filled with medical waste, because it cannot be rested on a surface and if set down can leak its contents. More rigid structures have been proposed which can stand up by themselves, either when empty or full of medical waste. However, more rigid liners which are permanently in a maximally expanded condition present a problem for packaging and transport because they take up large amounts of space.
Crushable liners have been proposed in which the liner is crushed for packaging and uncrushed prior to use. However, if the liner is made from material which is sufficiently rigid to be self-supporting then it tends to extend away from the crushed position; accordingly they must be held in the maximally crushed position, usually by enclosure with a film wrapper, to retain the benefit of the reduction in length. Furthermore, the crushing operation increases the maximum circumference of the liner as it tends to flatten so that it becomes wider than the vacuum jar aperture. Accordingly, the liner must be unpackaged and then straightened before it can be placed into a vacuum jar; these are unwanted secondary steps.

It has been proposed in GB 2333459 to provide a liner with a sidewall having concertina folds so that it can be moved to a collapsed configuration to shorten the axial length of the liner and reduce the amount of "dead space" within the liner for packaging and transport purposes. However, the concertina arrangement is not longitudinally rigid and therefore not resistant to compression and expansion as a vacuum is applied and released in use. Accordingly, the liner tends to be sucked up and down in the vacuum jar in use which is undesirable, not least because the volume of the liner is not constant. Furthermore, the concertina arrangement is at least partly biased towards its expanded condition and therefore it must be held in its collapsed configuration and prevented from expanding if the maximum benefit of its reduction in length is to be realised for packaging and transport.

The present invention seeks to address the problems with known medical suction disposal liners.
According to a first aspect of the present invention there is provided a medical suction disposal system liner comprising a flexible body having one or more longitudinally telescopic portions for allowing extension and/or shortening of the body, in which the body includes one or more fold lines defining the or each telescopic portions.

The present invention therefore utilises the general concept of telescoping such that parts of the body can slide into and/or out of one another to effect a reduction or extension in the effective length of the liner. The consequence of telescoping is that each telescopic portion can result in a reduction in length equal to its own length. Accordingly the number of telescopic portions required to result in a significant reduction in length is minimal. Therefore whereas large numbers of concertina folds are required to result in significant length reductions, the number of points of relative movement along the length of the body using telescopic portions is greatly reduced. As a result, the body of the present invention can be formed so as to be more resistant to extension and shortening in use under the influence of vacuum.

In a shortened position the or each telescopic portion may be defined by an internal longitudinal fold. In use, therefore, the body is unfolded into an extended position ready for use.

The body may comprise one or more longitudinal convolutions. The longitudinal convolutions can provide longitudinal folds in the body which define one or more telescopic portions which can be unfolded in a longitudinal translation movement.

The fold lines may comprise one or more circumferential fold regions. The fold regions may be adapted to facilitate folding, for example by use of regions of decreased wall thickness and/or shapes which permit folding.

In one embodiment the body comprises a fixed portion and a telescopic portion with the telescopic portion being connected to the fixed portion by a central rolling seal portion. The body therefore comprises three sections defined by two fold lines such that the telescopic portion is internal to the fixed portion with the outer wall of the telescopic portion adjacent the inner wall of the fixed portion. The central seal portion lies between the telescopic portion and the fixed portion and allows the telescopic portion to be longitudinally translated by a rolling action in which the fold lines become unfolded.

The body may comprise a plurality of concentric cylindrical portions. In one embodiment a fixed portion comprises the cylindrical portion with the greatest diameter, a central portion has a reduced diameter and a telescopic portion has the smallest diameter to allow the telescopic portion and the central portion to be folded into the fixed portion. Accordingly, in the shortened position the fixed portion represents the widest part of the body.

The body may be formed in a shortened position and movable, in use, to an extended position. By forming the body in the shortened position it is ready to be packaged and transported without the further need for secondary operations involving shortening the body and holding it in the shortened position. Alternatively the body may be formed in an extended position and movable to a shortened position for transport.

The body may be stable when in a shortened end position. In other words, when in the most shortened position the body does not tend to extend in the absence of some deliberate force. Accordingly, the body can be formed in or moved to a shortened end position and no further operation is required in order to retain it in the shortened position for packaging and transport.

The body may be stable when in an extended end position. In other words, when the body is maximally extended it does not tend to contract and shorten in length. The body is therefore less likely to contract in use as vacuum pressure is applied and released.

The body may be substantially tubular. A substantially cylindrical body with a circular section is a standard configuration for this type of liner, although other shapes, such as square or other polygonal shapes, may be preferred.

The body may be formed from a plastics material. Plastics materials are particularly useful because they can be moulded or otherwise formed into a required shape. The choice of plastics material and the thickness of the body wall can be used to change the properties of the liner. Polyethylene is a particularly useful material because it can be incinerated without yielding dangerous chemicals such as dioxins.

The body may be self-supporting in an extended position. In other words, in the extended position the body can stand upright. The body can therefore be placed down onto a surface temporarily even when full if required.

The body may comprise an open end and a closed end joined by a sidewall. The closed end may form a base member upon which the body can stand.

A cap with suction liner and vacuum connections may be provided for fitting to one end of the body, typically to the open end of a tubular body opposite a closed end. The liner may be supplied with the cap fitted to avoid the need to attach the cap before use.

If the liner is pre-fitted with a cap the attachment needs to be strong and stable to resist forces acting on the join when a vacuum is applied in use.

The liner may be welded to the cap to provide a strong joint. The weld may be effected by a sonic welding operation. However, laser welding has been found to produce a particularly neat finish and is a relatively quick process.

If laser welding is to be used then the interface between the liner and the cap prior to welding must be free from any gaps, because laser welding will not be effective across any gaps. Accordingly the liner and cap may be formed to provide a tapered fit so that the liner can be pushed onto the cap and the joint will tighten as it moves up the taper.

The tapered joint may be provided by forming tapering surfaces on either or both of the liner and the cap.

The extent of the taper may be in the region of 5° away from the longitudinal axis of the liner and/or cap. It has been found by the applicant that a 10° taper is too great. The laser welding may be effected by a Rofin DL x 25L laser with a wavelength of 808nm (+/- 10nm).

The liner may be adapted to fit in a standard vacuum jar in a shortened position. The telescopic principle of the present invention allows the body to assume a shortened configuration without increasing its dimensions transverse the longitudinal axis. Therefore it can be formed so as to be insertable into a vacuum jar in a shortened position and extended into the jar under the action of the vacuum when it is applied.

According to a second aspect of the present invention there is provided a method of forming a liner for use in a medical disposal system, comprising the steps of:
forming a flexible body; and forming one or more longitudinal folds in the body to form one or more longitudinally telescopic portions.

According to a third aspect of the present invention there is provided a method of forming a liner for use in a medical suction disposal system, comprising the step of forming a flexible tubular body having one or more longitudinal convolutions defining one or more telescopic portions.

By forming a tubular body with longitudinal convolutions which define one or more telescopic portions, the body can be formed in a shortened position. Thereafter the longitudinal convolutions are unfolded as the telescopic portion/s are longitudinally translated to allow the body to assume an extended position.

The body may be formed by moulded, such as blow moulding, injection moulding or vacuum forming.

The method may further comprise the step of fitting an end cap with suction liner and vacuum connections to the body. A cap may, for example, be heat sealed, ultrasonically welded or laser welded to an open end of a tubular bag-like body.

The present invention will now be more particularly described, by way of example, with reference to the accompanying drawings, in which like reference numerals refer to like features and in which:
Figure 1 is a half-section of a liner formed according to an embodiment of the present invention shown in an extended position;
Figure 2 is a perspective view of the liner of Figure 1;
Figure 3 is a perspective view of the liner of Figures 1 and 2 shown in a shortened position;
Figure 4 is a section of the liner shown in Figure 3;
Figure 5 is an under plan view of the liner shown in Figure 4;
Figures 6A to 6C illustrate the collapsing of the liner from the extended position shown in Figures 1 and 2 to the shortened position shown in Figures 3 to 5;
Figures 7 is a section of a liner formed according to an alternative embodiment;
Figure 8 is a section of the liner shown in Figure 7 taken along line VIII-VIII;
Figure 9 is a half-section of the liner shown in Figures 7 and 8 shown fitted with an end cap and placed in a vacuum jar, with the liner shown in a folded, shortened position;
Figure 10 is a half-section of the liner shown in Figure 9 in a partially unfolded position;
Figure 11 is a half-section of the liner shown in Figure 10 with the liner in a fully unfolded, extended position;
Figure 12 is a perspective view of a liner formed according to an alternative embodiment shown in an extended position;
Figure 13 is a side view of the liner of Figure 12;
Figure 14 is a perspective view of the liner shown in Figures 12 and 13 shown in a shortened, contracted condition;
Figure 15 is a section of the liner of Figure 13 taken along line XV-XV;
Figures 16A to 16C illustrate the expansion of the liner of Figures 12 to 15 from the contracted end position shown in Figure 14 to the extended end position shown in Figures 12 and 13;
Figure 17 is a side view of an end cap for use in conjunction with the liner of Figures 12 to 16;
Figure 18A is a section of the end cap of Figure 17 shown fitted to the liner of Figures 12 to 16; and
Figure 18B is a magnified section of the area circled in Figure 18A illustrating a laser weld.

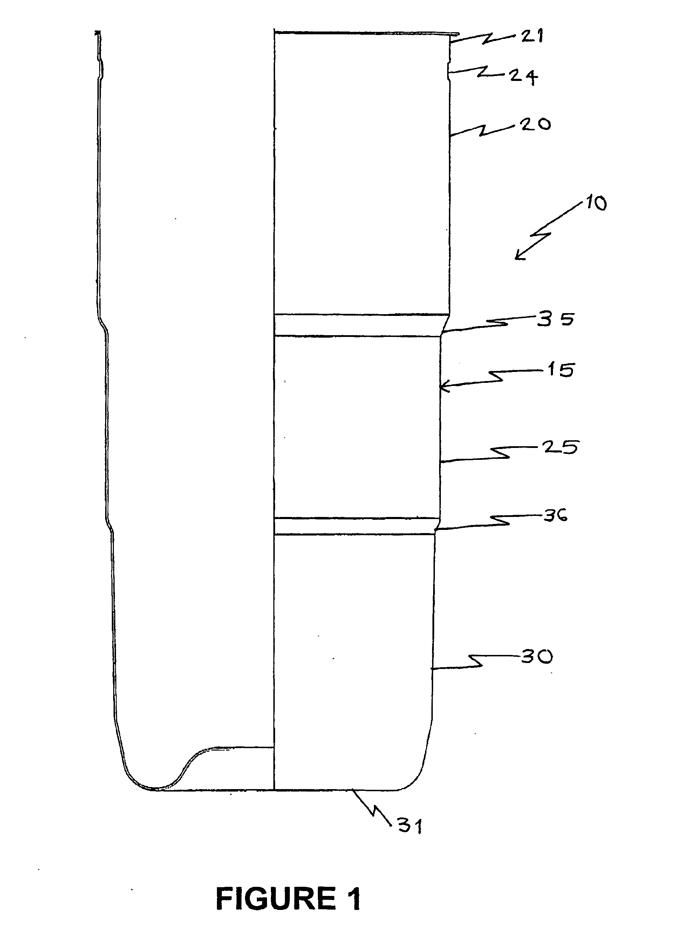

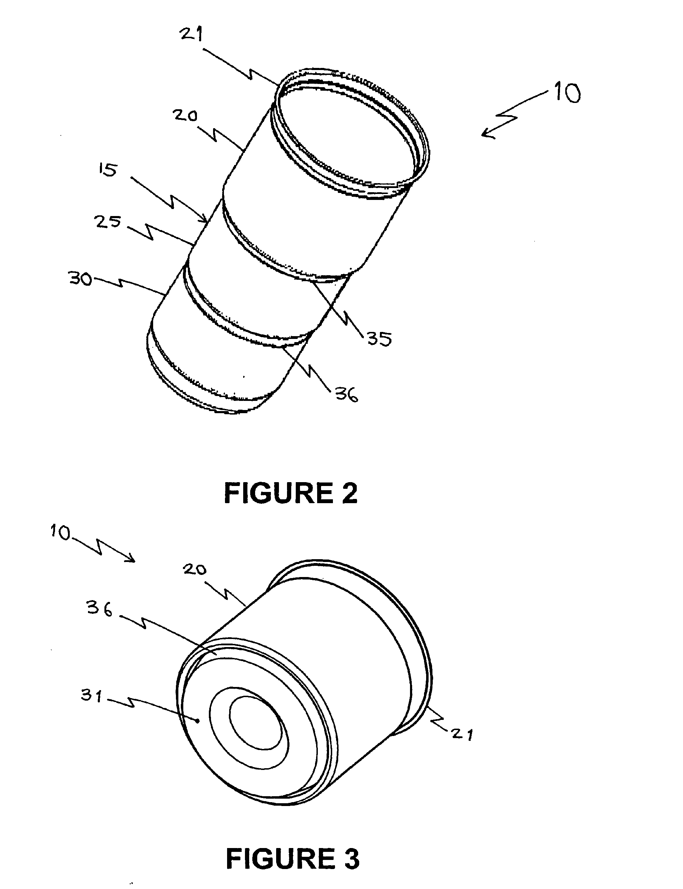

Referring first to Figures 1 and 2 there is shown a disposable liner generally indicated 10 for use in a medical suction disposal system.

The liner 10 comprises a generally cylindrical flexible body 15 having a circular cross-section around its longitudinal axis. In this embodiment the body 15 is formed from a low density polyethylene by blow moulding.

The body 15 comprises three main sections: an upper fixed portion 20 defining an open mouth 21; a central rolling section 25 which is contiguous with the fixed portion 20; and a cup-like telescopic portion 30 which is contiguous with the rolling portion 25 and includes an inwardly domed base 31 defining a closed end of the body 15 opposite the mouth 21.

The fixed portion 20, rolling portion 25 and telescopic portion 30 are concentric, with the fixed portion 20 having the largest diameter and the telescopic portion 30 having the smallest diameter. The intersection between the fixed portion 20 and the rolling portion 25 is defined by a tapering strip in the form of a radially inclined circumferentially extending step 35. Similarly, the rolling portion 25 is connected to the telescopic portion 30 by a radially inclined circumferentially extending step 36. In some embodiments the steps 35, 36 are thinner than one or both of the walls of the portions flanking them, which assists in the folding operations described below.

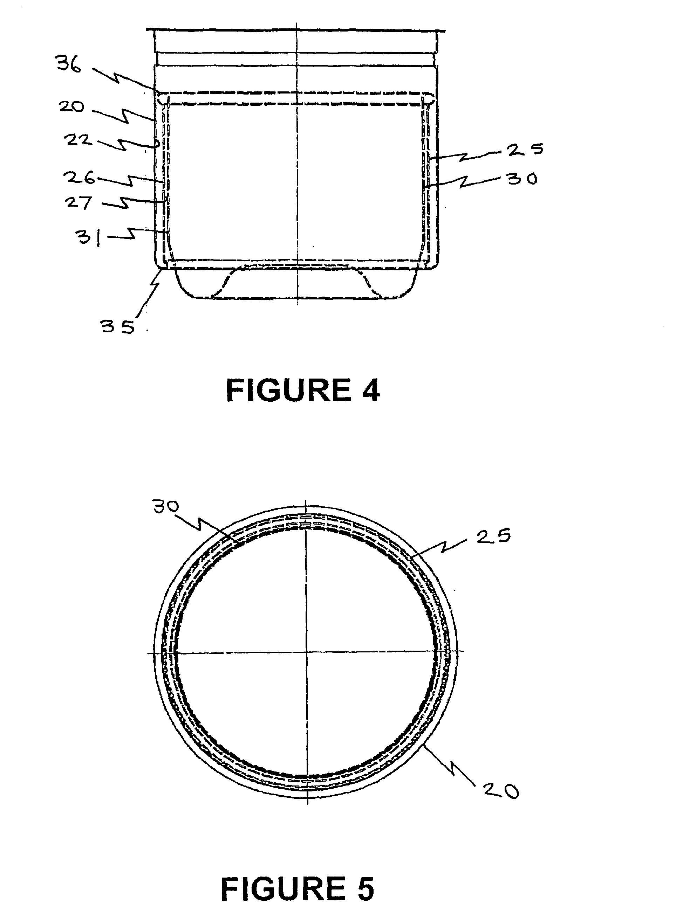

Referring now to Figures 3 to 5 the liner 10 is shown in a shortened configuration. As shown best in Figure 4, in the shortened position the telescopic portion 30 has been longitudinally translated to lie within the fixed portion 20. The translation is facilitated by the rolling portion 25 which is effectively turned inside-out. As a result, the exterior face 27 of the rolling portion 25 is parallel to and faces the exterior face 31 of the telescopic portion 30; and the interior face 26 of the rolling portion 25 is parallel to and faces the interior face 22 of the fixed portion 20. In this position the steps 35, 36 form longitudinal folds between the fixed portion 20 and the rolling portion 25 and the rolling portion 25 and the telescopic portion 30 respectively.

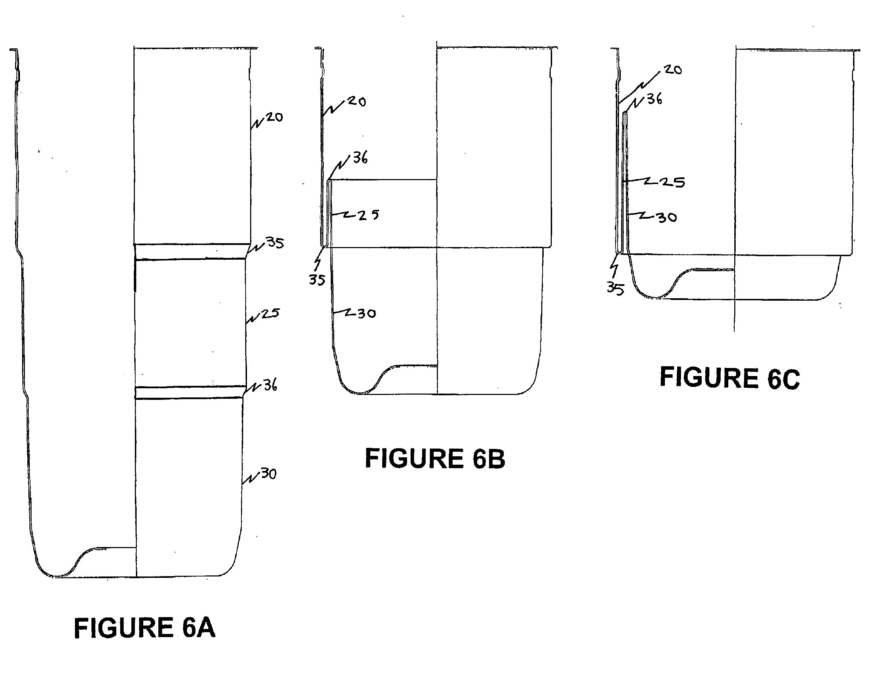

Referring now to Figures 6A to 6C the movement of the liner 10 from its extended position to its shortened position is illustrated.

In Figure 6A the liner is shown in its maximally extended position with the fixed portion 20, rolling portion 25 and telescopic portion 30 maximally spread along the longitudinal axis of the body 15.

With the fixed portion 20 remaining in position, the rolling portion 25 is upwardly translated. Beginning at the step 35 the portion 25 is progressively doubled over onto itself in a rolling action in which the position of a longitudinal fold along the length of the rolling portion 25 progressively moves towards the step 36 as illustrated in Figure 6B.

The rolling action of the rolling portion 25 continues until the step 36 is reached, at which point the rolling portion 25 has been turned inside-out within the fixed portion 20 and the telescopic portion 30 has been upwardly translated to lie at least partly within the axial length defined by the fixed portion 20. The rolling portion 25 moves longitudinally and thus could also be considered as a telescopic portion.

The steps 35, 36 comprise longitudinal folds in the body in which adjacent sections of the liner between the folds are parallel to each other and also parallel to the longitudinal axis of the body 15. The fold lines 35, 36 facilitate telescoping of the body 15.

It will be appreciated that by reversing the rolling action of the rolling portion 25 the telescopic portion 30 can be translated back to the position shown in Figure 6A via the position shown in Figure 6B.

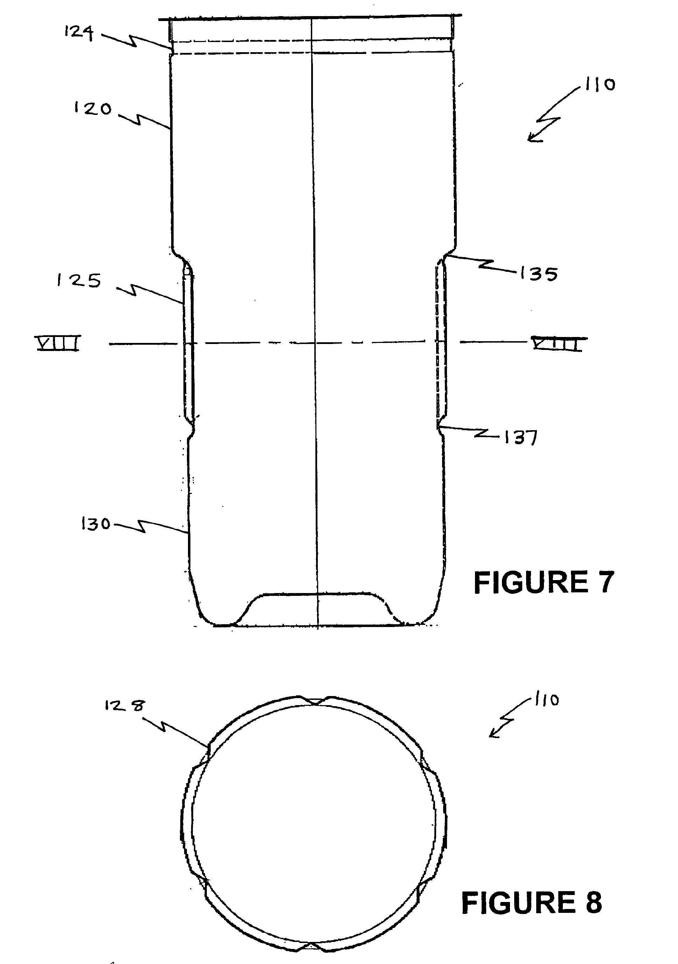

Referring now to Figures 7 and 8, there is shown a liner 110 formed according to an alternative embodiment. The liner 110 is similar to the liner 10 shown in Figures 1 to 6 in that it comprises a generally cylindrical body 115 with a fixed portion 120, a rolling portion 125 and a telescopic portion 130.

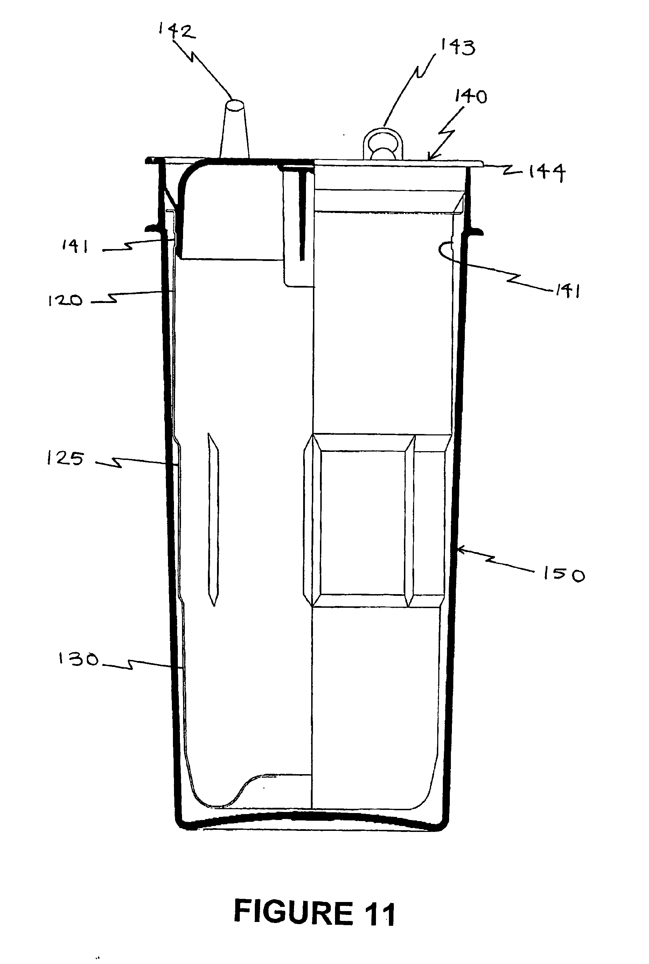

In this embodiment, the rolling section 125 is provided with a plurality of circumferentially spaced, longitudinally extending grooves 128 (see also figure 11). The grooves 128 allow the rolling portion 125 to circumferentially expand and contract as it is folded/unfolded in use.

The intersection between the fixed portion 120 and the rolling portion 125 is defined by an inwardly tapering circumferential step 135. The intersection between the rolling portion 125 and the telescopic portion 130 is defined by a circumferential groove 137. The groove 137 allows for longitudinal expansion and contraction of this region during folding and unfolding.

In this embodiment the thickness of the body 115 varies along its length. More specifically, the wall thickness of the fixed portion 115 is greater than that of the rolling portion 125 and the telescopic portion 130. This provides increased stiffness at the open end of the liner 110 and more flexibility in the folding region.

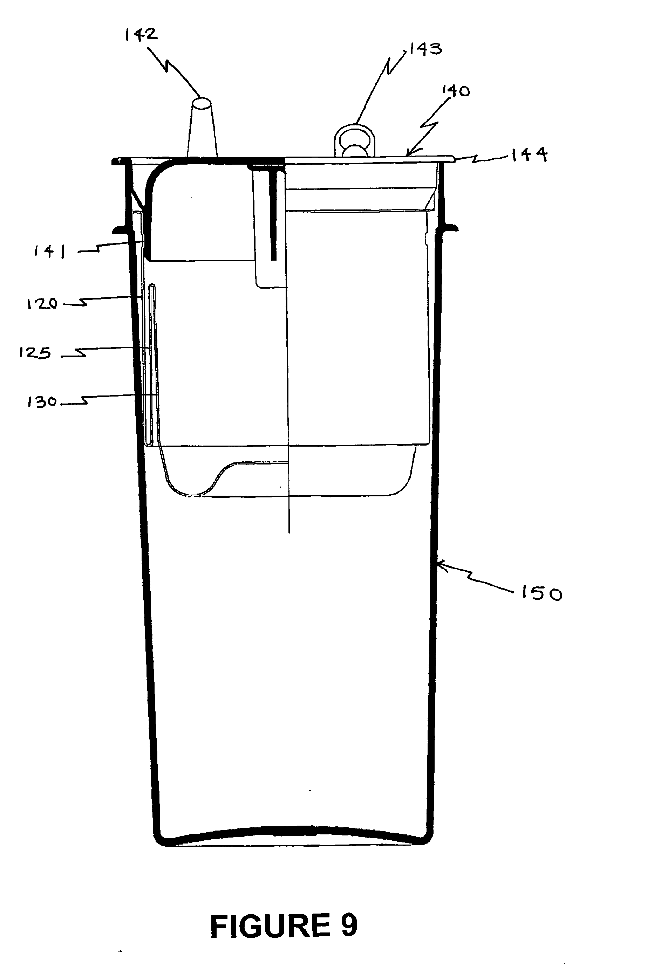

Referring now to Figures 9 to 11 the liner 110 shown in Figures 7 and 8 is shown in use.

The mouth 121 of the fixed portion 120 receives a conventional end cap 140.

The mouth 121 includes a circumferential recess 124 defining an internal bead which locates in a corresponding circumferential recess 141 on the cap. The cap 140 is joined to the liner 110 by heat sealing, or sonic welding in the region of the recess 141.

The cap 140 further comprises a suction line connection 142 and a vacuum connection 143. In addition, at the upper edge of the cap 140 is a circumferential flange 144 which allows the cap to be seated on the rim of a vacuum jar 150 in use.

The liner 110 is supplied fitted with the end cap 140 to the medical or other facility with the liner 110 in its collapsed/shortened configuration as shown in Figure 9. The liner 110 is fitted onto the vacuum jar 150 with the flange 144 of the cap 140 seated on the rim of the jar 150.

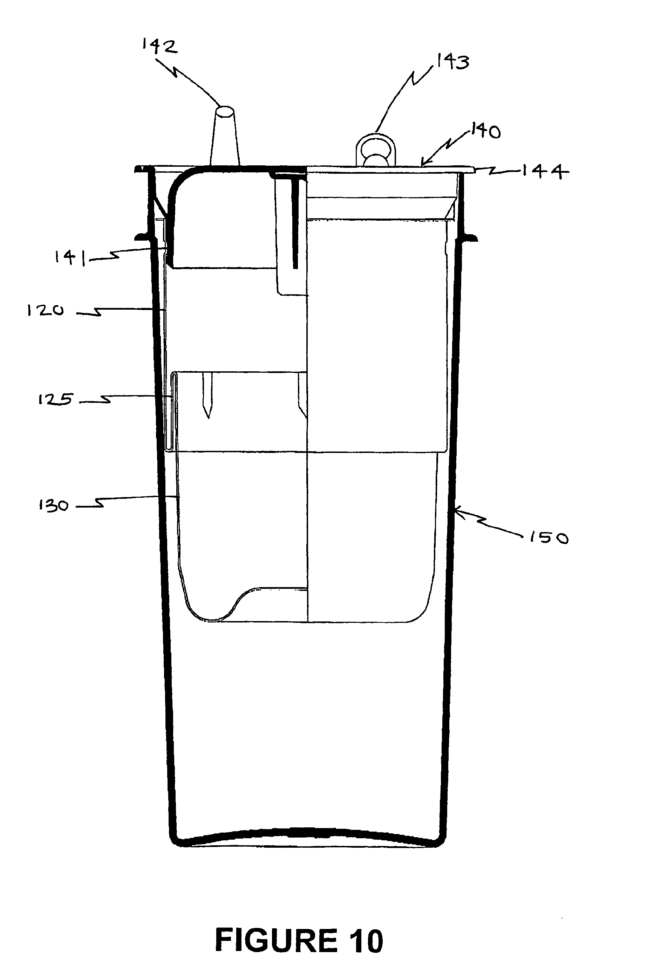

When a vacuum is applied the flange 144 seals onto the rim of the jar 150 and the vacuum in the jar around the liner 110 causes the telescopic portion 130 to be sucked down as shown progressively in Figures 10 and 11 to the maximally extended position shown in Figure 11.

In use the liner 110 is filled with medical waste. When the liner 110 is full the suction line connection 12 and vacuum connection 13 are sealed and the liner 110 can be removed from the vacuum jar 150. Thereafter the liner 110 is able to stand safely on the base 131. The liner 110 is stable in its extended position and therefore remains in this position when full of waste material. Furthermore, the resilience of the body 115 is sufficient to hold the liner 110 upright with no other support when it contains waste. Thereafter the liner 110 and its contents can be disposed of.

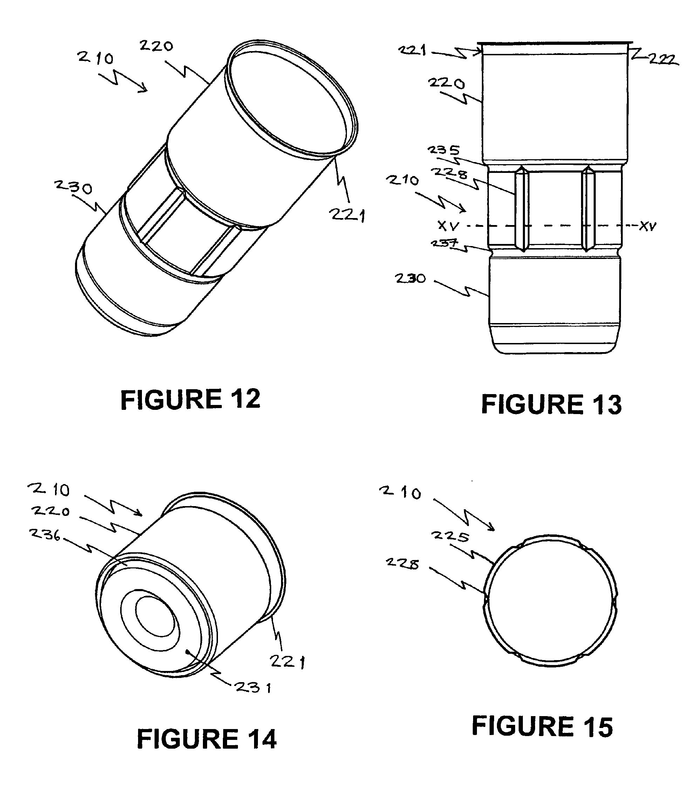

Referring now to Figures 12 to 15 there is shown a liner 210 formed according to an alternative embodiment.

The liner 210 is very similar to the liner 110 shown in Figures 7 to 11. The major exception is that the liner 210 does not comprise circumference recess for fitting it to an end cap. Instead, the mouth 221 terminates with a tapered section 222. The tapered section 222 is radially outwardly inclined by approximately 5° away from the longitudinal axis of the liner body 215.

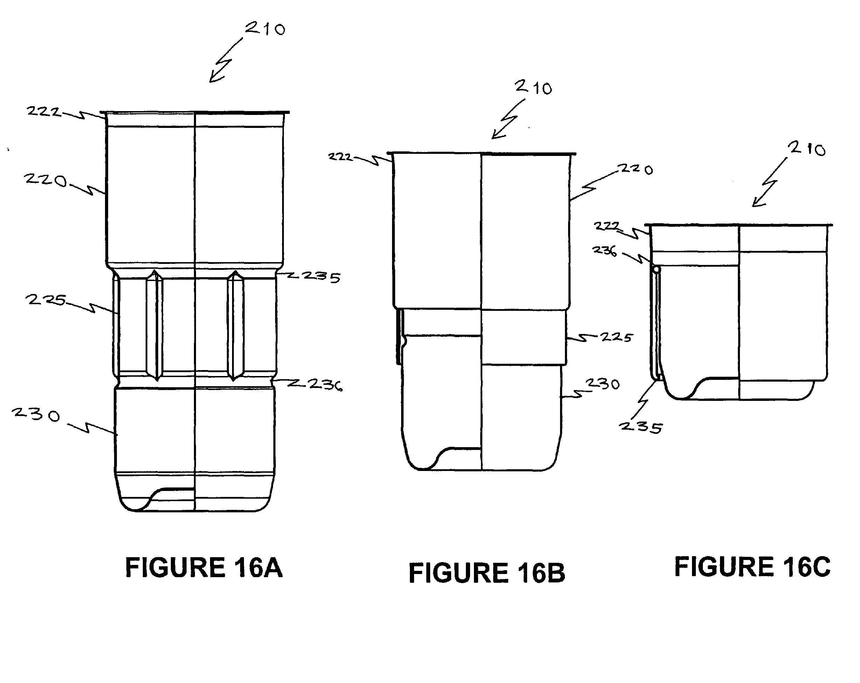

The liner 210 is formed in the extended position shown in Figure 16A but folded to the position shown in Figure 16C prior to use. In use the liner 210 is moved progressively from the position shown in Figure 16C to the position shown in Figure 16A via the position shown in Figure 16B. This process is the same as that described in relation to Figures 6A to 6C.

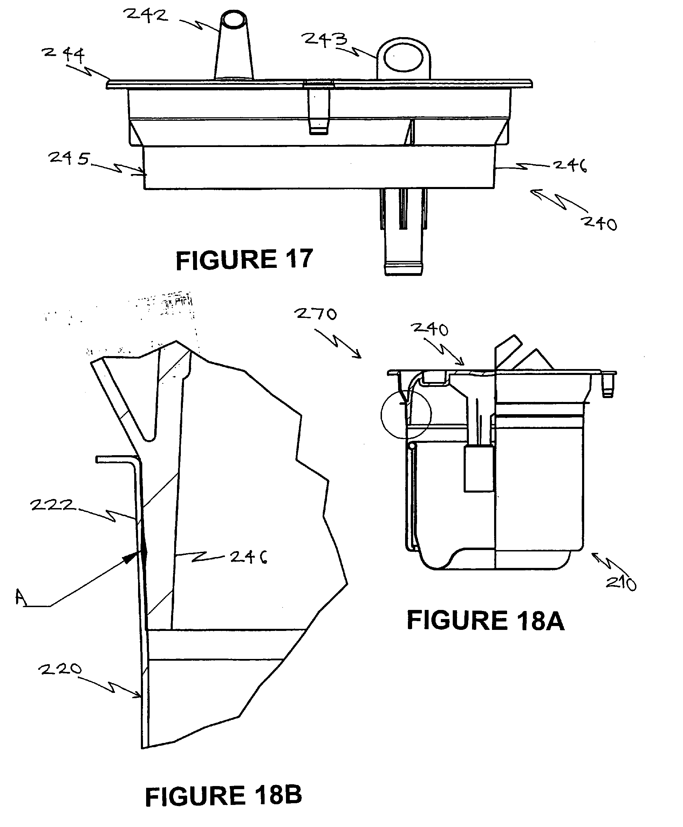

Referring now to Figure 17 there is shown an end cap 240 for use in conjunction with the liner 210 shown in Figures 12 to 16. The cap 240 is very similar to the cap 140 shown in Figures 9 to 11. The major exception is that the side shirt 245 of the cap 240 does not have a circumferential recess. Instead, the open end of the shirt 245 terminates with a tapered section 246. The tapered section 246 is radially outwardly inclined by approximately 5° away from the major axis of the cap 240, which corresponds to the tapering of the section 222 of the liner 210.

The skirt 245 is received in the tapered section 222 of the liner 210 and the liner 210 is pushed onto the cap 240 so that the tapered section 222 moves progressively up the tapered section 246. The result is that the interface between the cap 240 and the liner 210 is smooth and has no air gaps, as shown in Figures 18A and 18B. This allows the use of a circumferential laser weld to join the liner 210 to the cap 240 at point A.

The liner/cap assembly 270 shown in Figure 18A is in a stable form ready for use and can be transported in this collapsed state.

## Claims

1. A medical suction disposal system liner (10) comprising a flexible body (15) having one or more longitudinally telescopic portions (25,30) for allowing extension and/or shortening of the body, in which the body includes one or more fold lines (35,36) defining the or each telescopic portion.

2. A liner (10) as claimed in Claim 1, in which in a shortened position the or each telescopic portion (25,30) is defined by an internal longitudinal fold (35,36).

3. A liner (10) as claimed in Claim 1 or Claim 2, in which the body (15) comprises a fixed portion (20) and telescopic portion (30), the telescopic portion (30) being connected to the fixed portion by a rolling seal portion (25).

4. A liner (10) as claimed in any preceding Claim, in which the body (15) comprises a plurality of concentric cylindrical portions (20,25,30).

5. A liner (10) as claimed in any preceding Claim, in which the body (15) is formed in an extended position and is movable to a shortened position for transport and/or storage.

6. A liner (10) as claimed in any preceding Claim, in which in a shortened position the body (15) comprises one or more longitudinal convolutions (35,36).

7. A liner (10) as claimed in any preceding Claim, in which the body (15) is stable when in a shortened and/or extended end position.

8. A liner (10) as claimed in any preceding Claim, in which the body (15) is substantially tubular.

9. A liner (10) as claimed in any preceding Claim, in which the body (15) is formed from a plastics material.

10. A liner (10) as claimed in any preceding Claim, in which the body (15) is self-supporting in an extended position.

## Patentansprüche

1. Einsatz (10) eines Entsorgungssystems für medizinische Saugnäpfe bestehend aus einem flexiblen Körper (15) mit einem oder mehreren längslaufenden Teleskopteilen (25. 30), die eine Verlängerung und/oder Verkürzung des Körpers ermöglichen und in denen der Körper eine oder mehrere Faltlinien (35, 36) aufweist, welche jedes Teleskopteil begrenzen.

2. Einsatz (10) gemäß Anspruch 1, **dadurch gekennzeichnet, dass** jedes Teleskopteil (25, 30) in verkürzter Position von einer internen längslaufenden Faltung (35, 3) begrenzt wird.

3. Einsatz (10) gemäß Anspruch 1 oder 2, **dadurch gekennzeichnet, dass** der Körper (15) ein feststehendes Teil (20) und ein Teleskopteil (30) aufweist, wobei das Teleskopteil (30) durch eine Rolldichtung (25) mit dem feststehenden Teil verbunden ist.

4. Einsatz (10) gemäß einem der vorhergehenden Ansprüche, **dadurch gekennzeichnet, dass** der Körper (15) mehrere konzentrische zylinderförmige Teile (20, 25, 30) aufweist.

5. Einsatz (10) gemäß einem der vorhergehenden Ansprüche, **dadurch gekennzeichnet, dass** der Körper (15) in ausgefahrener Position gebildet ist und für Transport und/oder Lagerung in eine verkürzte Position verschoben werden kann.

6. Einsatz (10) gemäß einem der vorhergehenden Ansprüche, **dadurch gekennzeichnet, dass** der Körper (15) in verkürzter Position eine oder mehrere längslaufende Windungen (35, 36) aufweist.

7. Einsatz (10) gemäß einem der vorhergehenden Ansprüche, **dadurch gekennzeichnet, dass** der Körper (15) in verkürzter und/oder verlängerter Position stabil ist.

8. Einsatz (10) gemäß einem der vorhergehenden Ansprüche, **dadurch gekennzeichnet, dass** der Körper (15) im Wesentlichen röhrenförmig ausgebildet ist.

9. Einsatz (10) gemäß einem der vorhergehenden Ansprüche, **dadurch gekennzeichnet, dass** der Körper (15) aus einem Kunststoffmaterial gebildet ist.

10. Einsatz (10) gemäß einem der vorhergehenden Ansprüche, **dadurch gekennzeichnet, dass** der Körper (15) in verlängerter Position selbsttragend ist.

## Revendications

1. Gaine (10) de dispositif d'évacuation d'aspiration médicale comprenant un corps flexible (15), qui possède une ou plusieurs portions longitudinalement télescopique(s) (25, 30) étant apte(s) à permettre une extension et/ou un raccourcissement du corps, dans laquelle le corps comprend une ou plusieurs lignes de pli (35, 36) définissant la ou chaque portion télescopique.

2. Gaine (10) selon la revendication 1 dans laquelle, dans une position raccourcie, la ou chaque portion télescopique (25, 30) est définie par un pli longitudinal interne (35, 36).

3. Gaine (10) selon la revendication 1 ou la revendication 2, dans laquelle le corps (15) comprend une portion fixe (20) et une portion télescopique (30), la portion télescopique (30) étant connectée avec la portion fixe par une portion d'obturateur à roulement (25).

4. Gaine (10) selon l'une des revendications précédentes, dans laquelle le corps (15) comprend une pluralité de portions cylindriques concentriques (20, 25, 30).

5. Gaine (10) selon l'une des revendications précédentes, dans laquelle le corps (15) est formé dans une position en extension et est mobile jusqu'à une position raccourcie pour son transport et / ou son entreposage.

6. Gaine (10) selon l'une des revendications précédentes dans laquelle, dans une position raccourcie, le corps (15) comprend une ou plusieurs convolutions longitudinales (35, 36).

7. Gaine (10) selon l'une des revendications précédentes, dans laquelle le corps (15) est stable lorsqu'il est dans une position de fin de course raccourcie et / ou en extension.

8. Gaine (10) selon l'une des revendications précédentes, dans laquelle le corps (15) est essentiellement tubulaire.

9. Gaine (10) selon l'une des revendications précédentes, dans laquelle le corps (15) est formé dans un matériau plastique.

10. Gaine (10) selon l'une des revendications précédentes, dans laquelle le corps (15) est autoportant dans une position en extension.
